# EUROPÄISCHE PATENTANMELDUNG

(11) **EP 1 008 591 A1**
(43) Veröffentlichungstag der Anmeldung: **14.06.2000**
(21) Anmeldenummer: 99123202.6
(22) Anmeldetag: 25.11.1999
(51) Int. Cl.: C07D 277/78, C07D 277/36

(54) **Verfahren zur Herstellung von Dithiazolyldisulfiden**

(30) Priorität: 08.12.1998 DE 19856439
(71) Anmelder: Bayer Aktiengesellschaft, 51368 Leverkusen (DE)
(72) Erfinder: Nolte, Wilfried, Dr., 51519 Odenthal (DE); Köningshofen, Heinrich, Dr., 51465 Bergisch Gladbach (DE); Sicheneder, Adolf, Dr., 25551 Hohenlockstedt (DE)

(57) **Zusammenfassung**

Gegenstand der vorliegenden Erfindung ist ein Verfahren zur Herstellung von Dithiazolyl-(2,2')-disulfiden der allgemeinen Formel 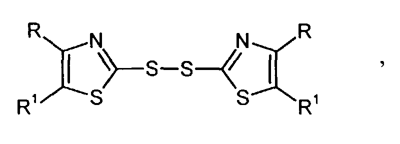 wobei
- R und R¹: gleich oder verschieden sein können und jeweils für Wasserstoff, Halogen, Nitro, Hydroxyl oder gegebenenfalls seinerseits substituiertes C₁-C₁₂-Alkyl oder Alkoxyl oder C₆-C₁₂-Cycloalkyl oder Aryl stehen oder gemeinsam den Rest 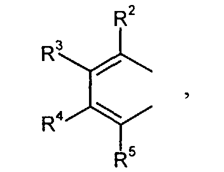 wobei
R² bis R⁵ die gleiche Bedeutung wie R und R¹ haben,
bilden, dadurch gekennzeichnet, daß man ein entsprechend substituiertes 2-Mercaptothizol mit Sauerstoff oder einem sauerstoffhaltigen Gas in Gegenwart eines Lösungsmittels und eines tertiären Amins und einer organischen Eisenverbindung oxidiert.

## Beschreibung

Die Erfindung betrifft ein neues Verfahren zur Herstellung von Dithiazolyl-(2,2')-disulfiden durch Oxidation von 2-Mercaptothiazolen, insbesondere von Dibenzothiazyldisulfid aus 2-Mercaptobenzthiazol, die so erhältliche Dithiazolyl-(2,2')-disulfide und deren Verwendung als Vulkanisationsbeschleuniger

Bei der technischen Herstellung des Dibenzothiazyldisulfiden durch Oxidation von 2-Mercaptobenzthiazolen wurden bislang verschiedene Oxidationsmittel verwendet (Ullmanns Encyclopedia of Industrial Chemistry, 5^{th} ed. Vol. A-26, p. 773-8, VCH, Weinheim, Basel, Cambridge, New York, Tokyo, 1995). So ist die Umsetzung mit Natriumchlorat und Natriumnitritlösung in salzsaurem Medium bei 30°C Stand der Technik. Dieses Verfahren ist mit einer Reihe von Nachteilen behaftet. Der Verbrauch an Mineralsäure ist sehr hoch (3 Mole HCl je Mol 2-Mercaptobenzthiazol) und es entstehen große Mengen an Beiprodukten. Weiterhin ist es bekannt, die Oxidation des 2-Mercaptobenzthiazolen mittels salpetriger Säure durchzuführen. Nach dem Verfahren der US-PS 19 08 935 wird 2-Mercaptobenzthiazol in Wasser suspendiert, ein wasserlösliches Nitrit zugefügt und der Sauerstoff oder ein sauerstoffhaltiges Gas wie Luft durch das Reaktionsgemisch geleitet. Gleichzeitig wird eine Mineralsäure, welche aus dem Nitrit salpetrige Säure freisetzt, zugefügt. Beim Verfahren gemäß US 21 19 131 und US 3 062 825 werden stöchiometrische Mengen Nitrit als alleiniges Oxidationsmittel eingesetzt. Hierdurch wird eine raschere und vollständigere Umsetzung erzielt. Diese Oxidationsverfahren sind insofern ebenfalls nachteilig, weil auch hier der Verbrauch an Mineralsäure sehr hoch ist und Salze sowie Stickoxide in großen Mengen als Beiprodukte anfallen.

Auch Chlor wurde bereits als Oxidationsmittel eingesetzt (Kirk-Othmer, Encyclopedia of Polymer Science and Technology (1970), Vol. 12, S. 262). Hierbei handelt es sich jedoch um eine komplizierte Reaktion mit kritischen Reaktionsbedingungen bei der häufig große Mengen an überoxidierten Nebenprodukten anfallen. Nach DE 23 09 584 werden zwecks Erhöhung der Produktausbeute und Verminderung der für eine ausreichende Oxidation benötigten Menge an überschüssigem Chlor unter der Oberfläche der Flüssigkeit unter starkem Rühren kontinuierlich getrennte Ströme aus einer wäßrigen Lösung eines Alkalimetallsalzes von Mercaptobenzthiazol, einer wäßrigen Lösung eines Alkalimetallhydroxids und gasförmiges Chlor bei 20 bis 75° miteinander umgesetzt, wobei der pH-Wert und das Redoxpotential der wäßrigen Mischung durch Regelung des Zuflusses der wäßrigen Hydroxidlösung und des gasförmigen Chlors bei pH 7 bis 10 und einem Redoxpotential von -150 bis 250 mV gehalten werden. Auch dieses Verfahren bedarf einer sehr sorgfältigen Steuerung um die Weiteroxidation des Dibenzothiazyldisulfids zu Benzothiazyl-2-sulfinat und -sulfonat zu verhindern. Nachteilig ist das Verfahren auch deshalb, weil große Mengen Alkalihydroxid verbraucht und große Mengen Kochsalz als Beiprodukt entstehen.

Hydroperoxide wie Wasserstoffperoxid, Alkalihydroperoxide und Aralkylhydroperoxide wurden ebenfalls bereits als Oxidationsmittel bei der Herstellung von Dibenzothiazyldisulfid eingesetzt (z.B. DE 23 49 314).

Allen obengenannten Oxidationsverfahren gemeinsam ist der Nachteil, daß vergleichsweise teure Oxidationsmittel und Säuren, Basen oder andere Hilfsstoffe benötigt werden, zum Teil auch nichtverwertbare Bei- oder Nebenprodukte anfallen.

Zu erwähnen ist auch noch ein Verfahren der elektrolytischen Oxidation von 2-Mercaptobenzol zu Dibenzothiazyldisulfid (DE 27 43 629).

Es wurde auch bereits untersucht, ob die Oxidation des 2-Mercaptobenzthiazols zu Dibenzothiazyldisulfid mit Sauerstoff als alleinigem Oxidationsmittel durchgeführt werden kann. Nach dem Verfahren gemäß US 3 654 297 ist dies möglich, wenn man als Katalysator ein Kobaltphthalocylaminsulfat, -disulfonat, -trisulfonat oder - tetrasulfonat oder Gemische derselben als Katalysator einsetzt und die Oxidation in einem organischen Lösungsmitel, welches weniger als 15 Gew.-% Wasser enthält bei Temperaturen von 50 bis 80°C, durchführt (vgl. auch Su 575 348; Chem. Abstr. 88 (1978), 89657 g). Die Herstellung und industrielle Anwendung dieses Katalysators sind jedoch problematisch.

Schließlich ist es aus DE 23 55 897 bekannt, die Oxidation des 2-Mercaptobenzthiazolen zu Dibenzothiazyldisulfiden unter gemeinsamer Verwendung von Sauerstoff oder einem Sauerstoff enthaltenden Gas und Eisenchlorid, insbesondere Eisen(III)-chlorid in einem gesättigten aliphatischen Alkohol mit 1 bis 10 Kohlenstoffatomen bei Temperaturen zwischen 0 und 150°C vorzunehmen. Dieser Katalysator führt jedoch nur dann zu einer befriedigenden Reaktionsgeschwindigkeit, wenn er in größerer Menge angewendet wird, nämlich im Verhältnis von 0,8 bis 1,5 Mol je Mol 2-Mercaptobenzthiazol. Der entscheidende Nachteil des Verfahrens ist jedoch, daß das Eisen während der Reaktion in Form basischer Salze ausfällt und das erhältliche Dibenzothiazyldisulfid stark mit Eisen verunreinigt ist. Ein auf diese Weise erhaltenes Produkt kann beispielsweise nicht ohne aufwendige Reinigung als Vulkanisationsmittel verwendet werden.

Gemäß DE 29 44 225 und DE 31 18 298 werden Schwermetall-Katalysatoren und Amine als Katalysatormischung eingesetzt, wobei das Amin auch durch Ammoniak ersetzt werden kann.

Die Verwendung von toxischen Schwermetall-Katalysatoren, wie Cd, ist aus ökologischen Gesichtspunkten problematisch, die Reste der toxischen Schwermetall-Katalysatoren sind zudem auch im endgültigen Produkt zu finden oder müssen aufwendig entfernt werden.

Es bestand daher nach wie vor das Bedürfnis zur Schaffung eines Verfahrens zur katalytischen Oxidation von 2-Mercaptobenzthiazolen mittels Sauerstoff oder sauerstoffhaltiger Gase.

Gegenstand der vorliegenden Erfindung ist ein Verfahren zur Herstellung von Dithiazolyl-(2,2')-disulfiden der allgemeinen Formel 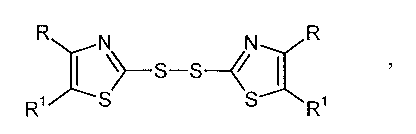 wobei
- R und R¹: gleich oder verschieden sein können und jeweils für Wasserstoff, Halogen, Nitro, Hydroxyl oder gegebenenfalls seinerseits sustituiertes C₁-C₁₂-Alkyl oder Alkoxyl oder C₆-C₁₂-Cycloalkyl oder Aryl stehen oder gemeinsam den Rest 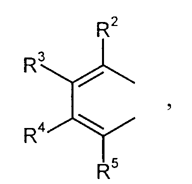 wobei
- R² bis R⁵: die gleiche Bedeutung wie R und R¹ haben,
bilden, dadurch gekennzeichnet, daß man ein entsprechend substituiertes 2-Mercaptothiazol mit Sauerstoff oder einem sauerstoffhaltigen Gas in Gegenwart eines Lösungsmittels und eines tertiären Amins und einer organischen Eisenverbindung oxidiert.

Falls die Reste R-R⁵ für Halogen stehen, so versteht der Fachmann hierunter Fluor, Chlor, Brom oder Jod, bevorzugt sind Chlor und Brom.

Unter C₁-C₁₂-Alkyl werden sämtliche dem Fachmann bekannte lineare oder verzweigte Alkylreste mit 1 bis 12 C-Atomen verstanden, wie Methyl, Ethyl, n-Propyl, i-Propyl, n-Butyl, i-Butyl, t-Butyl, n-Pentyl, i-Pentyl, neo-Pentyl und Hexyl, die ihrerseits wiederum substituiert sein können. Als Substituenten kommen hierbei Halogen, Nitro, Hydroxyl, oder auch C₁-C₁₂-Alkyl oder Alkoxy, sowie C₆-C₁₂-Cycloalkyl oder -Aryl in Frage, wie Benzoyl, Trimethylphenyl, Ethylphenyl, Chlormethyl, Chlorethyl und Nitromethyl.

Unter C₁-C₁₂-Alkoxyl werden sämtliche dem Fachmann bekannte lineare oder verzweigte Alkoxylreste mit 1 bis 12 C-Atomen verstanden, wie Methoxy, Ethoxy, n-Propoxy, i-Propoxy, n-Butoxy, i-Butoxy, t-Butoxy, n-Pentoxy, i-Pentoxy, neo-Pentoxy und Hexoxy, die ihrerseits wiederum substituiert sein können. Als Substituenten kommen hierbei Halogen, Nitro, Hydroxyl, oder auch C₁-C₁₂-Alkyl oder Alkoxyl, sowie C₆-C₁₂-Cycloalkyl oder -Aryl in Frage.

Unter C₆-C₁₂-Cycloalkyl werden sämtliche dem Fachmann bekannte ein- oder mehrkernige Cycloalkylreste mit 6 bis 12 C-Atomen verstanden, wie Cyclohexyl, Cycloheptyl, Cyclooctyl und Cyclononyl, die ihrerseits wiederum substituiert sein können. Als Substituenten kommen hierbei Halogen, Nitro, Hydroxyl, oder auch C₁-C₁₂-Alkyl oder Alkoxyl, sowie C₆-C₁₂-Cycloalkyl oder -Aryl in Frage, wie Methylcyclohexyl, Chlorcyclohexyl und Nitrocyclohexyl.

Unter C₆-C₁₂-Aryl werden sämtliche dem Fachmann bekannte ein- oder mehrkernige Arylreste mit 6 bis 12 C-Atomen verstanden, wie Phenyl, Naphthyl, die ihrerseits wiederum substituiert sein können. Als Substituenten kommen hierbei Halogen, Nitro, Hydroxyl, oder auch C₁-C₁₂-Alkyl oder Alkoxyl, sowie C₆-C₁₂-Cycloalkyl oder -Aryl in Frage, wie Bromphenyl, Chlorphenyl, Toloyl und Nitrophenyl.

Bevorzugt stehen die Reste R-R⁵ für Wasserstoff, Methyl, Ethyl, Propyl, t-Butyl, Methoxy, Ethoxy, Cyclohexyl, Benzoyl, Methoxy, Ethoxy, Phenyl, Naphthyl, Chlorphenyl, Toloyl und Nitrophenyl.

Dithiazolyl-(2,2')-disulfide werden als Vulkanisiermittel für Gummi verwendet. Das erfindungsgemäße Verfahren ist insbesondere für die Herstellung von Dibenzothiazolyl-(2,2')-disulfid, dem wichtigsten Vertreter dieser Verbindungsklasse, bedeutsam. Es ist jedoch ebenso mit Erfolg bei der Herstellung weiterer Verbindungen dieses Typs geeignet. Für die bevorzugte Herstellung von Dibenzothiazolyl-(2,2')-disulfid gelangt 2-Mercaptobenzthiazol als Ausgangsstoff zur Anwendung. Beispiele anderer 2-Mercaptothiazole, welche sich als Ausgangsstoffe für die Herstellung weiterer Dithiazolyl-(2,2')-disulfide der allgemeinen Formel (I) eignen, sind u.a. die in DE 23 55 897 genannten Verbindungen, u.a.
2-Mercaptothiazol
2-Mercapto-4-methylthiazol
2-Mercapto-4-ethylthiazol
2-Mercapto-4n-propylthiazol
2-Mercapto-4n-butylthiazol
2-Mercapto-4,5-dimethylthiazol
2-Mercapto-4,5-di-n-butylthiazol
2-Mercapto-4-phenylthiazol
2-Mercapto-5-chlor-4-phenylthiazol
2-Mercapto-4-p-bromphenylthiazol
2-Mercapto-4-m-nitrophenylthiazol
2-Mercapto-4-m-chlorphenylthiazol
2-Mercapto-4-methylbenzothiazol
2-Mercapto-5-methylbenzothiazol
2-Mercapto-6-methylbenzothiazol
2-Mercapto-4,5-dimethylbenzothiazol
2-Mercapto-4-phenylbenzothiazol
2-Mercapto-4-methoxybenzothiazol
2-Mercapto-6-methoxybenzothiazol
2-Mercapto-5,6-dimethoxybenzothiazol
2-Mercapto-6-methoxy-4-nitrobenzothiazol
2-Mercapto-6-ethoxybenzothiazol
2-Mercapto-4-chlorbenzothiazol
2-Mercapto-5-chlorbenzothiazol
2-Mercapto-6-chlorbenzothiazol
2-Mercapto-7-chlorbenzothiazol
2-Mercapto-5-chlor-6-methoxybenzothiazol
2-Mercapto-5-chlor-4-nitrobenzothiazol
2-Mercapto-5-chlor-6-nitrobenzothiazol
2-Mercapto-4,5-dichlorbenzothiazol
2-Mercapto-4,7-dichlorbenzothiazol
2-Mercapto-5-nitrobenzothiazol
2-Mercapto-6-nitrobenzothiazol
2-Mercapto-4-phenylbenzothiazol
2-Mercapto-naphthothiazol
2-Mercapto-6-hydroxybenzothiazol

Als Oxidationsmitel wird Sauerstoff oder ein sauerstoffhaltiges Gas, vorzugsweise Luft verwendet. Umsatz und Selektivität steigen mit zunehmendem Sauerstoffdruck bzw. Partialdruck. Im allgemeinen liegt beim erfindungsgemäßen Verfahren der Sauerstoffdruck bzw. -partialdruck im Bereich von 0,1 bis 150 bar. Aus ökonomischen Gründen werden vorzugsweise Sauerstoffdrücke bzw. -partialdrücke von 2 bis 10 bar angewendet, besonders bevorzugt von 3 bis 8 bar.

Als Lösungsmittel eignen sich alle oxidationsstabilen organischen Lösungsmittel. Beispiele hierfür sind Alkohole, Dimethylformamid, Benzol, Toluol und Chlorbenzol. Geeignete Alkohole sind beispielsweise aliphatische Alkohole mit 1 bis 10 Kohlenstoffatomen, insbesondere Methanol, Ethanol, Propanol, Isopropanol, n-Butanol, sec.-Butanol, tert.-Butanol, Pentanol, Hexanol, Heptanol und Octanol. Vorzugsweise werden Toluol und Isopropanol angewendet. Weiterhin geeignet ist Wasser, welches besonders bevorzugt als Lösungsmitel im erfindungsgemäßen Verfahren eingesetzt wird. Die Konzentration des Lösungsmittels ist nicht kritisch. Im allgemeinen liegt die Lösungsmittelmenge im Bereich von 200 bis 1200 Gew.-%, bezogen auf eingesetztes 2-Mercaptothiazol. Größere Mengen Lösungsmittel sind aus ökonomischen Gründen zu vermeiden, da in diesen Fällen auch größere Mengen tertiäres Amin benötigt werden.

Geeignete tertiäre Amine sind aliphatische, cycloaliphatische, aromatische und heterocyclische Amine wie beispielsweise Trimethylamin, Triethylamin, Tri-n-propylamin, Tri-n-butylamin, n-Octyldimethylamin, Diisopropyl-ethylamin, Propyl-dimethylamin, Ethyl-dimethylamin, Isopropyl-dimethylamin, Butyl-dimethylamin, Pyridin, N-Methyl-pyridin, N-Methylpyrrolidin, 2,4,6-Trimethyl-pyridin, 2,3,4,5-Tetramethyl-pyridin, 2,3,4,5,6-Penta-methylpyridin, Dimethylanilin, Dimethylbenzylamin (DMBA), 4-Methylamino-pyridin und 1,4-Diazabicyclo-(2,2,2)-octan. Bevorzugte tertiäre Amine sind Trimethylamin, Triethylamin, Tri-n-propylamin, Tri-n-butylamin und Dimethylbenzylamin. Besonders bevorzugt ist Dimethylbenzylamin.

Die genannten tertiären Amine sind nicht alle gleich stark wirksam. In der Regel ist die katalytische Wirksamkeit umso größer, je höher die Basizität des Amins ist. Daneben können aber auch sterische Effekte die katalytische Wirksamkeit des tertiären Amins mitbeeinflussen.

Die Menge des tertiären Amins kann in einem weiten Bereich variiert werden. Ausreichend sind bereits katalytische Mengen. Mit steigenden Mengen des tertiären Amins steigt auch dessen katalytische Wirksamkeit, wobei es mehr auf die Konzentration des tertiären Amins im Reaktionsgemisch und weniger auf das Mengenverhältnis von tertiärem Amin zu dem eingesetzten 2-Mercaptothiazol ankommt. Vorzugsweise wird das tertiäre Amin in Mengen von 0,1 bis 20 Gew.-%, bezogen auf das Reaktionsgemisch, eingesetzt.

Die erfindungsgemäß anzuwendenden tertiären Amine können sowohl alleine als auch gemeinsam mit einer Hydroxid-Verbindung eingesetzt werden.

Als Hydroxidverbindung kommen Alkali- und Erdalkalimetallhydroxide und Ammoniumhydroxid, wie Lithiumhydroxid, Natriumhydroxid, Kaliumhydroxid, Magnesiumhydroxid und Calciumhydroxid in Frage.

Bevorzugt sind Natriumhydroxid, Kaliumhydroxid und Ammoniumhydroxid.

Die verwendeten Mengen an Hydroxidverbindung liegen im Bereich von 0,001 bis 5 Mol pro Mol Thiazol, bevorzugt 0,05 bis 2 Mol, besonders bevorzugt 0,09 bis 1,5 Mol.

Als organische Eisenverbindung eignen sich sämtliche dem Fachmann bekannte Eisenkomplexe, in denen das Eisen mit hoher Komplexbildungskonstante komplexiert ist, wie Eisen(III)haemin, Eisen-hemiporphyrazin (Fe-hemi), Eisen-phthalocyanin, bevorzugt wird Eisen-hemiporphyrazin eingesetzt.

Die verwendeten Mengen an organischer Eisenverbindung liegen im Bereich von 0,01 bis 1000 mmol pro Mol Thiazol, bevorzugt 0,1 bis 100 mmol, besonders bevorzugt 0,5 bis 10 mmol.

Die Reaktionstemperatur beträgt 0 bis 150°C, vorzugsweise 20 bis 90°C und insbesondere 60 bis 80°C. Bei tieferen Temperaturen nimmt die Reaktionsgeschwindigkeit ab, bei höheren Temperaturen verringert sich die Selektivität der Reaktion.

Die Reaktionsdauer beträgt in der Regel 0,5 bis 30 Stunden, unter den genannten bevorzugten Druck- und Temperaturbedingungen.

Die Durchführung des erfindungsgemäßen Verfahrens erfolgt in einfacher Weise dadurch, daß der Sauerstoff bzw. das sauerstoffhaltige Gas unter den angegebenen Druck- und Temperaturbedingungen in/bzw. durch die aus dem 2-Mercaptothiazol, dem Lösungsmittel, organischer Eisenverbindung und tertiärem Amin bestehende Lösung geleitet wird. Da gegebenenfalls unumgesetztes 2-Mercaptothiazol im Lösungsmittel gelöst bleibt, gestaltet sich die Aufarbeitung des Reaktionsgemisches sehr einfach. Das ausgefallene Reaktionsprodukt wird abfiltriert oder abgeschleudert, die Mutterlauge mit frischem 2-Mercaptothiazol versetzt und im Kreislauf geführt. Je nachdem wie hoch die Anfangskonzentration der organischen Eisenverbindung war, muß nach einer bestimmten Anzahl von Reaktionszyklen frische organische Eisenverbindung zugefügt werden. Außerdem sollte vorzugsweise dann das bei der Umsetzung entstandene Reaktionswasser aus der Mutterlauge ausgeschleust werden, wenn sein Gehalt - bezogen auf Mutterlauge - mehr als 10 Gew.-% beträgt.

Beim erfindungsgemäßen Verfahren werden praktisch quantitative Ausbeuten und Selektivitäten von mehr als 99 % erzielt. Die erhältlichen Dithiazolyl-(2,2)-disulfide zeichnen sich durch hohe Reinheit aus, sie können ohne weitere Reinigung beispielsweise direkt als Gummivulkanisationsmittel eingesetzt werden. Gegenüber den bekannten Verfahren, bei welchen ebenfalls ein 2-Mercaptobenzthiazol mittels Sauerstoffoxidiert wird, zeichnet sich das erfindungsgemäße Verfahren dadurch aus, daß einfache und billige Katalysatoren in sehr geringer Menge zur Anwendung gelangen und daß diese Katalysatoren mit der Mutterlauge im Kreislauf geführt werden können, ohne daß ihre Aktivität merklich nachläßt, ohne daß giftige Schwermetalle eingesetzt und aus dem Produkt wieder entfernt werden müssen.

Die erfindungsgemäß herstellbaren Dithiazolyl-(2,2')-disulfide eignen sich hervorragend als Vulkanisationsbeschleuniger in schwefelhaltigen Kautschukmischungen. Insbesondere geeignet ist das Dibenzothiazyldisulfid.

Die schwefelhaltigen Kautschukmischungen können auf übliche Weise hergestellt und vulkanisiert werden, wie beispielsweise näher in Encyclopedia of Polymer Science and Engineering, Vol. 4, S. 66 ff (Compoundierung) und Vol. 17, S. 666 ff (Vulkanisation) beschrieben wird.

Die schwefelhaltigen Kautschukmischungen können selbstverständlich noch weitere Kautschukhilfsprodukte enthalten, wie Reaktionsbeschleuniger, Alterungsschutzmittel, Wärmestabilisatoren, Lichtschutzmittel, Ozonschutzmittel, Verarbeitungshilfsmittel, Weichmacher, Tackifier, Treibmittel, Farbstoffe, Pigmente, Wachse, Streckmittel, organische Säuren, Verzögerer, Metalloxide sowie Aktivatoren wie Triethanolamin, Polyethylenglykol, Hexantriol, die in der Gummiindustrie bekannt und üblich sind. Die Kautschukhilfsmittel werden in üblichen Mengen zugemischt und richten sich nach dem jeweils beabsichtigten Verwendungszweck. Übliche Mengen sind beispielsweise Mengen von 0,1 bis 50 Gew.-%, bezogen auf die Gesamtmenge des eingesetzten Kautschuks. Bevorzugt werden zur Verbesserung der Hitze-und Lagerstabilität bekannte phenolische, aminische, schwefelhaltige oder phosphorhaltige Alterungsschutzmittel zugegeben. Diese sind z. B. in Ullmanns Enzyklopädie der technischen Chemie, Band 8, S. 19 ff näher beschrieben.

Neben den zuvor erwähnten Kautschukhilfsprodukten können den erfindungsgemäßen Kautschukmischungen die bekannten Vernetzer zugegeben werden, wie Schwefel, Schwefelspender oder Peroxide. Die erwähnten Vernetzer werden üblicherweise in Mengen von 0,1 bis 10 Gew.-%, bevorzugt 0,1 bis 5 Gew.-%, bezogen auf die Gesamtmenge des jeweils eingesetzten Kautschuks, eingesetzt.

Die Vulkanisation der erfindungsgemäßen Kautschukmischungen kann bei üblichen Temperaturen von 100 bis 200°C, bevorzugt 130 bis 180°C (gegebenenfalls unter Druck 10 bis 200 bar), erfolgen.

Die Abmischung der Kautschuke mit den anderen erwähnten Kautschukhilfsprodukten, Vernetzern und Beschleunigern kann in üblicher Weise mit Hilfe von geeigneten Mischaggregaten, wie Walzen, Innenmischer und Mischextruder, durchgeführt werden.

### Beispiele

### Beispiel 1

In einem Autoklaven werden 600 ml Wasser, 83,6 g (0,5 mol) 2-Mercaptobenzthiazol (MBT), 13,5 g (0,1 mol) Dimethylbenzylamin (DMBA) und 0,3 g (0,5 mmol) Eisen-hemiporphyrazin bei einem Sauerstoffpartialdruck von 6 bar vorgelegt und unter Rühren bei 60°C umgesetzt. Nach 16,5 h Reaktion wurden 143 g Dibenzothiazyldisulfid (MBTS) abfiltriert.

### Beispiel 2

In einem Autoklaven wurden 600 g Methanol, 100,3 g (0,6 mol) MBT, 22,68 g (0,168 mol) DMBA und 0,36 g (0,6 mmol) Eisen-hemiporphyrazin vorgelegt. Anschließend wurde 4 h lang bei 60°C 10 bis 15 l/h Luft durch die Mischung geleitet. Man erhielt 180 g MBTS, die abfiltriert werden konnten.

### Beispiel 3

Es wurde analog Beispiel 2 gearbeitet, jedoch wurde anstelle von DMBA 17 g (0,168 mol) Triethylamin eingesetzt. Man erhielt 98 g MBTS, die abfiltriert werden konnten.

### Beispiel 4

Es wurde analog Beispiel 3 gearbeitet, jedoch wurde anstelle von Eisen-hemiporphyrazin 0,34 g (0,6 mmol) Eisenphthalocyanin eingesetzt. Man erhielt 33,8 g MBTS, die abfiltriert werden konnte.

### Beispiel 5

In einem Autoklaven wurden 167 g (1 mol) MBT, 1 500 ml Wasser, 60,75 g (0,45 mol) DMBA und 8 g (0,2 mol) NaOH vorgelegt, der pH betrug 8,3. Anschließend wurden 3,5 h lang bei 60°C 10 bis 15 l/h Luft durch die Mischung geleitet. Man erhielt 46,5 g MBTS, die abfiltriert werden konnten.

### Beispiel 6

Es wurde analog Beispiel 5 gearbeitet, jedoch auf Zugabe von NaOH verzichtet und stattdessen die Menge an DMBA auf 837 g (6,2 mol) erhöht, der pH betrug 7,8. Man erhielt 37,2 g MBTS, die abfiltriert werden konnten.

## Patentansprüche

1. Verfahren zur Herstellung von Dithiazolyl-(2,2')-disulfiden der allgemeinen Formel 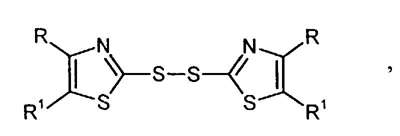 wobei
R und R¹ gleich oder verschieden sein können und jeweils für Wasserstoff, Halogen, Nitro, Hydroxyl oder gegebenenfalls substituiertes C₁-C₁₂-Alkyl oder -Alkoxyl oder C₆-C₁₂-Cycloalkyl oder -Aryl stehen oder gemeinsam den Rest 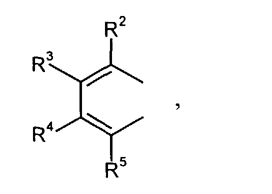 wobei
R² bis R⁵ die gleiche Bedeutung wie R und R¹ haben,
bilden, dadurch gekennzeichnet, daß man ein entsprechend substituiertes 2-Mercaptothiazol mit Sauerstoff oder einem sauerstoffhaltigen Gas in Gegenwart eines Lösungsmittels und eines tertiären Amins sowie einer organischen Eisenverbindung oxidiert.

2. Verfahren gemäß Anspruch 1, dadurch gekennzeichnet, daß man als Lösungsmittel Wasser einsetzt.

3. Verfahren gemäß Anspruch 1, dadurch gekennzeichnet, daß man als tertiäres Amin Diethylbenzylamin verwendet.

4. Verfahren gemäß Anspruch 1, dadurch gekennzeichnet, daß man das tertiäre Amin in Mengen im Bereich von 0,001 bis 5 Mol pro Mol Thiazol einsetzt.

5. Verfahren gemäß Anspruch 1, dadurch gekennzeichnet, daß man als organische Eisenverbindung Eisenhemiporphyrazin einsetzt.

6. Verfahren gemäß Anspruch 1, dadurch gekennzeichnet, daß man die organische Eisenverbindung in Mengen im Bereich von 0,01 bis 1000 mmol pro Mol Thiazol einsetzt.

7. Verfahren zur Herstellung von Dibenzothiazolyl-(2,2')-disulfid, dadurch gekennzeichnet, daß man 2-Mercaptobenzthiazol mit Sauerstoff oder einem sauerstoffhaltigen Gas in Gegenwart von Wasser und einem tertiären Amin und Eisenhemiporphyrazin oxidiert.

8. Dithiazolyl-(2,2')-disulfide herstellbar nach einem Verfahren gemäß Anspruch 1.

9. Verwendung der Dithiazolyl-(2,2')-disulfide gemäß Anspruch 8 als Vulkanisationsbeschleuniger in Kautschukmischungen.
